(19) Europäisches Patentamt
European Patent Office
Office européen des brevets

(11) EP 1 428 844 A2

(12) **DEMANDE DE BREVET EUROPEEN**

(43) Date de publication:
**16.06.2004 Bulletin 2004/25**

(21) Numéro de dépôt: **03293120.6**

(22) Date de dépôt: **12.12.2003**

(51) Int Cl.[7]: **C08F 265/04**, C08F 265/00, C08F 290/04, C08L 51/00, A61K 7/00

(84) Etats contractants désignés:
**AT BE BG CH CY CZ DE DK EE ES FI FR GB GR HU IE IT LI LU MC NL PT RO SE SI SK TR**
Etats d'extension désignés:
**AL LT LV MK**

(30) Priorité: **12.12.2002 FR 0215738**
**12.12.2002 FR 0215737**

(71) Demandeur: **L'OREAL**
**75008 Paris (FR)**

(72) Inventeur: **Lion, Bertrand**
**95270 Luzarches (FR)**

(74) Mandataire: **Dodin, Catherine**
**L'OREAL - D.I.P.I.**
**25-29 Quai Aulagnier**
**92600 Asnières (FR)**

(54) **Dispersions de polymères en milieu organique.**

(57) L'invention a trait à des dispersions de particules autostabilisées de polymères dans un milieu non aqueux non siliconé, et à un procédé de préparation desdits polymères.

L'invention concerne également des compositions cosmétiques ou pharmaceutiques comprenant ladite dispersion de particules de polymères.



Printed by Jouve, 75001 PARIS (FR)

## Description

La présente invention a trait à des dispersions stables de particules formées par des polymères acryliques dans un milieu non aqueux et non siliconé, ainsi qu'à l'utilisation de ces dispersions dans des compositions cosmétiques et aux compositions ainsi obtenues.

Il est connu d'utiliser dans le domaine de la cosmétique des dispersions de particules de polymère dans des milieux organiques, en tant qu'agents filmogènes dans différentes formulations de cosmétique, telles que les mascaras, les eye-liners, les ombres à paupières ou les vernis à ongles.
Ainsi, dans la demande de brevet européen EP-A-0749747, il est décrit une composition comprenant une dispersion de particules de polymères non solubles dans un milieu non aqueux, ladite dispersion étant stabilisée par ajout de polymères stabilisants. Les polymères stabilisants selon ce document se lient de manière non covalente par le biais d'interactions physiques sur les polymères non solubles évoqués ci-dessus.
Toutefois, ce type de composition présente les inconvénients suivants : elle nécessite l'ajout dans le milieu non aqueux d'une quantité de polymères dits stabilisants supérieure à celle effectivement liée aux particules de polymères non solubles, afin d'obtenir une dispersion desdites particules relativement stable. Or, lors de l'ajout dans ces compositions d'adjuvants, tels que des pigments, une partie des polymères stabilisants a tendance à se désorber des particules de polymères non solubles pour s'associer avec lesdits adjuvants, ce qui contribue à déstabiliser la dispersion, notamment par formation d'agglomérats entre les particules de polymères.
Le document JP 11181003 décrit des polymères aptes à former des particules solides sans ajout de polymères stabilisants mais toutefois ces particules sont instables dans des milieux organiques non aqueux.

La demanderesse a découvert de manière surprenante de nouveaux polymères, aptes à former des particules stables dans un milieu non aqueux non siliconé, sans ajout de polymères stabilisants.

Ainsi, la présente invention a pour but de proposer une dispersion, dans un milieu organique non aqueux et non siliconé, de particules individuelles autostabilisées de polymères, ladite dispersion étant exempte d'agglomérats de particules et de sédiments insolubles, visuellement, par exemple après mise au repos de la dispersion une journée (24 heures) à température ambiante (20°C).

Un premier objet de la présente invention est donc une dispersion dans un milieu organique non aqueux non siliconé de particules notamment solides constituées d'au moins un polymère acrylique comprenant un squelette insoluble dans ledit milieu, et une partie soluble dans ledit milieu constituée de chaînes latérales liées de manière covalente audit squelette, ledit polymère étant susceptible d'être obtenu par polymérisation radicalaire dans ledit milieu :

- d'au moins un monomère acrylique, pour former ledit squelette insoluble ; et
- d'au moins un macromonomère carboné comportant un groupe terminal apte à réagir pendant la polymérisation pour former les chaînes latérales, ledit macromonomère ayant une masse moléculaire en poids supérieure ou égale à 200 et représentant 1 à 18% en poids du polymère.

Un autre objet de l'invention est une composition cosmétique ou pharmaceutique comprenant, dans un milieu cosmétiquement ou pharmaceutiquement acceptable, une dispersion telle que ci-après définie.

Les dispersions selon l'invention sont donc exemptes de polymère stabilisant, tels que ceux décrits dans EP749747, et les polymères selon l'invention ne sont donc pas stabilisés en surface par de tels polymères stabilisants additionnels.

La dispersion selon l'invention comprend donc un milieu organique non aqueux non siliconé.
On entend, dans ce qui précède et ce qui suit, par "milieu organique non aqueux", un milieu constitué principalement d'un ou plusieurs composés organiques ou non, liquides tels que définis ci-après, ledit milieu pouvant contenir au plus 1% en poids d'eau.
On entend, dans ce qui précède et ce qui suit, par "milieu non siliconé" un milieu comprenant un ou plusieurs composés non siliconés, notamment organiques, tels que définis ci-après, lesdits composés non siliconés étant présents majoritairement, c'est-à-dire à au moins 50% en poids, notamment de 50 à 100% en poids, par exemple de 60 à 99% ou encore de 65 à 95% en poids, par rapport au poids total du milieu, c'est-à-dire du mélange 'composés siliconés éventuels + composés organiques non siliconés + eau éventuelle'.
Ledit milieu peut donc éventuellement comprendre des composés siliconés qui peuvent être présents en une quantité maximale de 50% en poids, notamment de 0 à 40% en poids, voire de 1 à 35% en poids, et encore de 5-30% en poids par rapport au poids total du milieu.
De préférence, le milieu non siliconé non aqueux est liquide.

Parmi les composés non aqueux non siliconés susceptibles d'être présents dans ledit milieu organique non aqueux non siliconé, on peut citer :

- les composés liquides non aqueux non siliconés ayant un paramètre de solubilité global selon l'espace de solubilité

de Hansen inférieur ou égal à 18 $(MPa)^{1/2}$, de préférence inférieur ou égal à 17 $(MPa)^{1/2}$,

- les monoalcools ayant un paramètre de solubilité global selon l'espace de solubilité de Hansen inférieur ou égal à 20 $(MPa)^{1/2}$ ; et
- les mélanges de ceux-ci.

Le paramètre de solubilité global δ selon l'espace de solubilité de Hansen est défini dans l'article « Solubility parameter values » de Eric A.Grulke de l'ouvrage « Polymer Handbook », 3éme édition, Chapitre VII, p.519-559 par la relation :

$$\delta = (d_D^{\ 2} + d_P^{\ 2} + d_H^{\ 2})^{1/2}$$

dans laquelle

- $d_D$ caractérise les forces de dispersion de LONDON issues de la formation de dipôles induits lors des chocs moléculaires,
- $d_P$ caractérise les forces d'intéractions de DEBYE entre dipôles permanents, et
- $d_H$ caractérise les forces d'intéractions spécifiques (type liaisons hydrogène, acide/base, donneur/accepteur, etc.).

La définition des solvants dans l'espace de solubilité selon Hansen est décrite dans l'article de C.M.Hansen « The three dimensional solubility parameters » J.Paint Technol. 39, 105 (1967).

Parmi les composés liquides non aqueux non siliconés ayant un paramètre de solubilité global selon l'espace de solubilité de Hansen inférieur ou égal à 18 $(Mpa)^{1/2}$, on peut citer des corps gras liquides, notamment des huiles, qui peuvent être choisies parmi les huiles naturelles ou synthétiques, carbonées, hydrocarbonées, fluorées, éventuellement ramifiées, seules ou en mélange.

Parmi ces huiles, on peut citer les huiles végétales formées par des esters d'acides gras et de polyols, en particulier les triglycérides, telles que l'huile de tournesol, de sésame ou de colza, ou les esters dérivés d'acides ou d'alcools à longue chaîne (c'est-à-dire ayant de 6 à 20 atomes de carbone), notamment les esters de formule RCOOR' dans laquelle R représente le reste d'un acide gras supérieur comportant de 7 à 19 atomes de carbone et R' représente une chaîne hydrocarbonée comportant de 3 à 20 atomes de carbone, tels que les palmitates, les adipates et les benzoates, notamment l'adipate de diisopropyle.

On peut également citer les alcanes linéaires, ramifiés et/ou cycliques éventuellement volatils et notamment des huiles de paraffine, de vaseline, ou le polyisobutylène hydrogéné, l'isododécane, ou encore les 'ISOPARS', les isoparaffines volatiles. On peut citer également les esters, les éthers, les cétones.

On peut citer, en particulier, comme composés liquides ayant un paramètre de solubilité global selon l'espace de solubilité de Hansen inférieur ou égal à 18 $(MPa)^{1/2}$ :

- les esters linéaires, ramifiés ou cycliques, ayant plus de 6 atomes de carbone, notamment 6 à 30 atomes de carbone;
- les éthers ayant plus de 6 atomes de carbone, notamment 6 à 30 atomes de carbone ; et
- les cétones ayant plus de 6 atomes de carbone, notamment 6 à 30 atomes de carbone.

Par monoalcools ayant un paramètre de solubilité global selon l'espace de solubilité de Hansen inférieur ou égal à 20 $(MPa)^{1/2}$, on entend les monoalcools gras aliphatiques ayant 6 à 30 atomes de carbone, la chaîne hydrocarbonée ne comportant pas de groupement de substitution. Comme monoalcools selon l'invention, on peut citer l'alcool oléique, le décanol, le dodécanol, l'octadécanol et l'alcool linoléique.

Parmi les composés siliconés éventuellement présents, en faible quantité, dans le milieu non aqueux non siliconé, on peut citer les huiles de silicone telles que les polydiméthylsiloxanes et les polyméthylphénylsiloxanes, éventuellement substitués par des groupements aliphatiques et/ou aromatiques, éventuellement fluorés, ou par des groupements fonctionnels tels que des groupements hydroxyles, thiols et/ou amines, et les huiles siliconées volatiles, notamment cycliques.

Le choix des monomères constituant le squelette du polymère, le choix du macromonomère, la masse moléculaire du polymère, du macromonomère ainsi que la proportion desdits macromonomères et des monomères se fera en fonction du milieu non aqueux et non siliconé de manière à obtenir une dispersion de particules solides de polymères stable dans ledit milieu, ce choix pouvant être effectué par l'homme du métier.

Par "dispersion stable", on entend, selon l'invention, une dispersion qui n'est pas susceptible de former de dépôt solide ou de déphasage liquide/solide notamment après une centrifugation, par exemple, à 4000 tours/minute pendant 15 minutes.

Les polymères acryliques formant les particules en dispersion comprennent donc un squelette insoluble dans ledit milieu et une partie soluble dans ledit milieu.
Ces polymères peuvent se présenter sous différentes formes, en particulier sous forme de polymères statistiques.
Selon l'invention, on entend par "polymère acrylique" un polymère susceptible d'être obtenu par polymérisation radicalaire :

- d'un ou plusieurs monomères acryliques, éventuellement en mélange avec un ou plusieurs monomères additionnels vinyliques non acryliques,
- avec un ou plusieurs macromonomères, dans un milieu organique non aqueux et non siliconé donné, ou dans un milieu de polymérisation autre.

De préférence, les monomères acryliques représentent 50-100% en poids, notamment 60-95% en poids, voire 70-90% en poids du mélange monomères acryliques + monomères vinyliques non acryliques éventuels.
De préférence, les monomères acryliques sont choisis parmi les monomères dont l'homopolymère est insoluble dans le milieu organique non aqueux considéré, c'est-à-dire que l'homopolymère est sous forme solide (ou non dissous) à une concentration supérieure ou égale à 5% en poids à température ambiante (20°C) dans ledit milieu organique non aqueux.
Selon l'invention, on entend par "macromonomère" tout polymère, notamment oligomère, comportant sur une seule de ses extrémités un groupe terminal, notamment polymérisable, apte à réagir lors de la réaction de polymérisation avec les monomères considérés, pour former les chaînes latérales, ledit groupe pouvant être avantageusement un groupe à insaturation éthylénique susceptible de se polymériser par voie radicalaire avec les monomères constituant le squelette.
De préférence, le macromonomère est choisi parmi les macromonomères dont l'homopolymère est soluble dans le milieu organique considéré, c'est-à-dire complètement dissous à une concentration supérieure ou égale à 5% en poids et à température ambiante dans ledit milieu organique non aqueux.
Ainsi, les polymères selon l'invention se présentent sous la forme de polymères insolubles dans le milieu considéré, et comprennent un squelette (ou chaîne principale) constitué par un enchaînement de motifs notamment acryliques résultant de la polymérisation notamment d'un ou plusieurs monomères acryliques et des chaînes latérales (ou greffons) issus de la réaction des macromonomères, lesdites chaînes latérales étant liées de manière covalente à ladite chaîne principale.
Le squelette (ou chaîne principale) est insoluble dans le milieu considéré alors que les chaînes latérales (ou greffons) sont solubles dans ledit milieu.
Comme monomère acrylique susceptible d'être employé pour constituer après polymérisation le squelette insoluble du polymère, on peut citer, seul ou en mélange, les monomères suivants, ainsi que leurs sels :

- (i) les (méth)acrylates de formule :

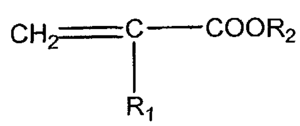

dans laquelle :

- $R_1$ désigne un atome d'hydrogène ou un groupe méthyle ;
- $R_2$ représente un groupe choisi parmi :
    - un groupe alkyle linéaire ou ramifié, comprenant de 1 à 6 atomes de carbone, ledit groupe pouvant comporter dans sa chaîne un ou plusieurs hétéroatomes choisis parmi O, N et S; et/ou pouvant comporter un ou plusieurs substituants choisis parmi -OH, les atomes d'halogène (F, Cl, Br, I) et
    - NR'R" avec R' et R" identiques ou différents choisis parmi les alkyles linéaires ou ramifiés en C1-C4; et/ou pouvant être substitué par au moins un groupe polyoxyalkylène, notamment polyoxyéthylène et/ou polyoxypropylène, ledit groupe polyoxyalkylène étant constitué par la répétition de 5 à 30 motifs oxyalkylène;
    - un groupe alkyle cyclique comprenant de 3 à 6 atomes de carbone, ledit groupe pouvant comporter dans sa chaîne un ou plusieurs hétéroatomes choisis parmi O, N et S, et/ou pouvant comporter un ou plusieurs substituants choisis parmi OH et les atomes d'halogène (F, Cl, Br, I).

A titre d'exemples de $R_2$, on peut citer le groupe méthyle, éthyle, propyle, butyle, isobutyle, méthoxyéthyle, éthoxyéthyle, méthoxy-polyoxyéthylène (350 OE); trifluoroéthyle; 2-hydroxyéthyle, 2-hydroxypropyle, diméthylaminoéthyle, diéthylaminoéthyle, diméthylaminopropyle.

- (ii) les (méth)acrylamides de formule :

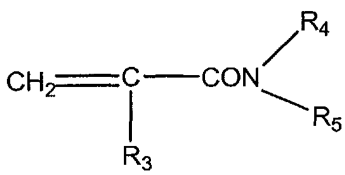

dans laquelle :

- $R_3$ désigne un atome d'hydrogène ou un groupe méthyle ;
- $R_4$ et $R_5$, identiques ou différents, représentent un atome d'hydrogène ou un groupe alkyle, linéaire ou ramifié, comportant de 1 à 6 atomes de carbone, pouvant comporter un ou plusieurs substituants choisis parmi -OH, les atomes d'halogène (F, Cl, Br, I) et -NR'R" avec R' et R" identiques ou différents choisis parmi les alkyles linéaires ou ramifiés en C1-C4;ou
- $R_4$ représente un atome d'hydrogène et $R_5$ représente un groupe 1,1-diméthyl-3-oxobutyle.

A titre d'exemples de groupes alkyles pouvant constituer $R_4$ et $R_5$, on peut citer n-butyle, t-butyle, n-propyle, diméthylaminoéthyle, diéthylaminoéthyle, diméthylaminopropyle.

- (iii) des monomères à insaturation(s) éthylénique(s) comprenant au moins une fonction acide carboxylique, phosphorique ou sulfonique, tel que l'acide acrylique, l'acide méthacrylique, l'acide acrylamidopropanesulfonique, et leurs sels;

Parmi ces monomères acryliques, on peut tout particulièrement citer les (méth) acrylates de méthyle, d'éthyle, de propyle, de butyle, d'isobutyle; les (méth)acrylates de méthoxyéthyle ou d'éthoxyéthyle; le méthacrylate de trifluoroéthyle; le méthacrylate de diméthylaminoéthyle, le méthacrylate de diéthylaminoéthyle, le méthacrylate de 2-hydroxypropyle, le méthacrylate de 2-hydroxyéthyle, l'acrylate de 2-hydroxypropyle, l'acrylate de 2-hydroxyéthyle ; le diméthylaminopropylméthacrylamide; et leurs sels.

Parmi les monomères additionnels vinyliques, on peut citer :

- les esters de vinyle de formule : $R_6$-COO-CH=$CH_2$
  dans laquelle $R_6$ représente un groupe alkyle linéaire ou ramifié, comprenant de 1 à 6 atomes, ou un groupe alkyle cyclique comportant de 3 à 6 atomes de carbone et/ou un groupe aromatique, par exemple de type benzénique, anthracénique, et naphtalénique ;
- des monomères à insaturation(s) éthylénique(s) comprenant au moins une fonction acide carboxylique, phosphorique ou sulfonique, tel que l'acide crotonique, l'anhydride maléique, l'acide itaconique, l'acide fumarique, l'acide maléique, l'acide styrènesulfonique, l'acide vinylbenzoïque, l'acide vinylphosphorique et les sels de ceux-ci ;
- les monomères à insaturation(s) éthylénique(s) comprenant au moins une fonction amine tertiaire, tel que la 2-vinylpyridine, la 4-vinylpyridine,
  et leurs mélanges.

Parmi les sels, on peut citer ceux obtenus par neutralisation des groupements acides à l'aide de base inorganiques telles que l'hydroxyde de sodium, l'hydroxyde de potassium, l'hydroxyde d'ammonium ou de bases organiques de type alkanols amines comme la monoéthanolamine, la diéthanolamine, la triéthanolamine, la 2-méthyl-2-amino-1-propanol.

On peut également citer ceux formés par neutralisation des motifs amine tertiaire, par exemple à l'aide d'acide minéral ou organique. Parmi les acides minéraux, on peut citer l'acide sulfurique ou l'acide chlorhydrique, l'acide bromhydrique, iodhydrique, l'acide phosphorique, l'acide borique. Parmi les acides organiques, on peut citer les acides comportant un ou plusieurs groupes carboxylique, sulfonique, ou phosphonique. Il peut s'agir d'acides aliphatiques linéaires, ramifiés ou cycliques ou encore d'acides aromatiques. Ces acides peuvent comporter, en outre, un ou plusieurs hétéroatomes choisis parmi O et N, par exemple sous la forme de groupes hydroxyles. On peut notamment citer l'acide acétique ou l'acide propionique, l'acide téréphtalique, ainsi que l'acide citrique et l'acide tartrique.

Il est entendu que ces monomères acryliques non polymérisés peuvent être solubles dans le milieu considéré, mais deviennent insolubles après polymérisation en une quantité appropriée, et forment alors une dispersion de particules solides dudit polymère, ce qui est l'objectif de la présente invention.

Les macromonomères constituant après réaction les chaînes latérales du polymère selon l'invention, comportent en bout de chaîne un groupe terminal apte à réagir au cours de la polymérisation avec les monomères acryliques et vinyliques, pour former lesdites chaînes, ledit groupe terminal polymérisable étant en particulier un groupe vinyle, de préférence un groupe (méth)acryloyloxy (acrylate ou méthacrylate).

Les macromonomères sont choisis préférentiellement parmi les macromonomères hydrocarbonés, et notamment parmi ceux dont les homopolymères présentent une température de transition vitreuse (Tg) inférieure ou égale à 25°C, notamment allant de -100°C à 25°C, de préférence allant de -80°C à 0°C inclus.
De préférence, les macromonomères selon l'invention présentent une masse moléculaire moyenne en poids (Mw) allant de 200 à 100 000, de préférence de 300 à 50 000, notamment 500 à 20 000, préférentiellement de 800 à 10000, voire de 1000 à 6000.

Comme macromonomères susceptibles d'être utilisés, on peut en particulier citer :

- (i) les homopolymères et les copolymères de (méth)acrylate d'alkyle linéaire ou ramifié en C6-C22, de préférence C8-C18, présentant un groupe terminal polymérisable choisi parmi les groupes vinyle ou (méth)acryloyloxy.
  De tels macromonomères sont notamment décrits dans les brevets EP895467 et EP96459 et dans l'article Gillman K.F., Polymer Letters, Vol 5, page 477-481 (1967).
  On peut citer en particulier les macromonomères de poly(acrylate d'éthyl-2 hexyle) à extrémité monoacrylate ou monométhacrylate; les macromonomères de poly(acrylate de dodécyle) ou de poly(méthacrylate de dodécyle) à extrémité monoacrylate ou monométhacrylate; les macromonomères de poly(acrylate de stéaryle) ou de poly(méthacrylate de stéaryle) à extrémité monoacrylate ou monométhacrylate.

- (ii) les polyoléfines ayant un groupe terminal à insaturation éthylénique de type vinyle ou (méth)acryloyloxy, de préférence (méth)acrylate parmi lesquelles on peut citer en particulier :

  - les macromonomères de polyéthylène, les macromonomères de polypropylène, les macromonomères de polyisobutylène, les macromonomères de polybutadiène; tous ces macromonomères étant à extrémité monoacrylate ou monométhacrylate;
  - les macromonomères de polyisoprène à extrémité monoacrylate ou monométhacrylate;
  - les macromonomères de poly(éthylène/butylène)-polyisoprène à extrémité monoacrylate ou monométhacrylate
  - les macromonomères de copolymère polyéthylène/polypropylène ou de copolymère polyéthylène/polybutylène, à extrémité monoacrylate ou monométhacrylate.

  De tels macromonomères sont en particulier décrits dans EP1347013 ou encore dans US5625005 qui mentionne des macromonomères éthylène/butylène et éthylène/propylène à groupement terminal réactif (méth)acrylate.

En particulier, ces macromonomères peuvent être représentés par la formule suivante :

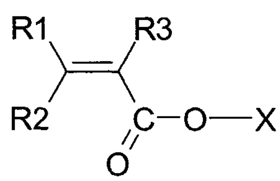

dans laquelle R1, R2 et R3, indépendamment l'un de l'autre, représentent l'hydrogène ou un groupe alkyle, linéaire, cyclique ou ramifié, ayant de 1 à 16 atomes de carbone, notamment 1 à 6; de préférence l'hydrogène;
et X représente une chaîne comprenant des motifs oxyde d'éthylène et/ou oxyde de propylène et/ou oxyde de butylène.

On peut en particulier citer le méthacrylate de poly(éthylène/butylène), tel que celui commercialisé sous la dénomination Kraton Liquid L-1253 par Kraton Polymers.

Les macromonomères sont de préférence présents dans les polymères de l'invention dans une proportion de 1 à 18% en poids, préférentiellement de 2 à 16% en poids, et notamment de 4 à 15% en poids, entre autre de 6 à 12% en poids, voire de 8 à10% en poids, par rapport au poids total dudit polymère.

Des polymères particulièrement avantageux selon l'invention sont ceux obtenus par polymérisation :

- du monomère acrylate de méthyle et d'un macromonomère polyéthylène/polybutylène à terminaison méthacrylate dans un solvant tel que l'isododécane, l'isononylisononanoate, l'octyldodécanol, le diisostéaryl malate ou un alkylbenzoate en $C_{12}$-$C_{15}$ (tel que le Finsolv TN);
- du monomère acrylate de méthoxyéthyle et d'un macromonomère polyéthylène/polybutylène à terminaison méthacrylate notamment dans l'isododécane;
- d'un mélange de monomères acrylate de méthyle / méthacrylate de méthyle et d'un macromonomère polyéthylène/ polybutylène à terminaison méthacrylate notamment dans l'isododécane;
- d'un mélange de monomères acrylate de méthyle / acide acrylique et d'un macromonomère polyéthylène/polybutylène à terminaison méthacrylate dans un solvant tel que l'isododécane;
- d'un mélange des monomères acrylate de méthyle / méthacrylate de diméthylaminoéthyle et d'un macromonomère polyéthylène/polybutylène à terminaison méthacrylate dans un solvant tel que l'isododécane;
- d'un mélange des monomères acrylate de méthyle / méthacrylate de 2-hydroxyéthyle et d'un macromonomère polyéthylène/polybutylène à terminaison méthacrylate en particulier dans l'isododécane.

De préférence, la masse moléculaire en poids (Mw) du polymère est comprise entre 10 000 et 300 000, notamment entre 20 000 et 200 000, mieux encore entre 25 000 et 150 000.

Grâce aux caractéristiques susmentionnées, dans un milieu organique non siliconé donné, les polymères ont la capacité de se replier sur eux-mêmes, formant ainsi des particules de préférence solides de forme sensiblement sphérique, avec sur le pourtour de ces particules les chaînes latérales déployées, qui assurent la stabilité de ces particules. De telles particules résultant des caractéristiques des polymères de l'invention, ont la particularité de ne pas s'agglomérer dans ledit milieu et donc de s'autostabiliser et de former une dispersion de particules de polymère particulièrement stable.

En particulier, les polymères de l'invention sont aptes à former des particules solides nanométriques, de taille moyenne allant de 10 à 400 nm, de préférence de 20 à 200 nm.

Du fait de cette taille très faible, les particules entrant dans la constitution de la dispersion sont particulièrement stables et donc peu susceptibles de former des agglomérats.

La dispersion de l'invention est donc une dispersion stable dans le milieu considéré et ne forme pas de sédiments, lorsqu'elle est placée pendant une durée prolongée (par exemple 24 heures) à température ambiante (25°C).

De préférence, la dispersion de particules présente un taux de matière sèche (ou extrait sec) de 40% à 70% en poids de matière sèche, notamment de 45 à 65% en poids.

On peut préparer ledit polymère ou ladite dispersion de particules de polymère, par un procédé comprenant une étape consistant à réaliser une copolymérisation radicalaire, dans un milieu répondant à la définition donnée précédemment, d'un ou plusieurs monomères acryliques tels que définis précédemment avec un ou plusieurs macromonomères tels que définis précédemment.

D'une manière classique, la copolymérisation peut être effectuée en présence d'un initiateur de polymérisation. Les initiateurs de polymérisation peuvent être des amorceurs radicalaires. De manière générale, un tel initiateur de polymérisation peut être choisi parmi les composés organiques peroxydés tels que le dilauroyl peroxyde, le dibenzoyl peroxyde, le tert-butyl peroxy-2-éthylhexanoate ; les composés diazotés tels que l'azobisisobutyronitrile, l'azobisdiméthylvalero-nitrile.

La réaction peut être également initiée à l'aide de photoinitiateurs ou par une radiation telle que des UV, des neutrons ou par plasma.

D'une manière générale, pour mettre en oeuvre ce procédé, on introduit, dans un réacteur de taille appropriée à la quantité de polymère que l'on va réaliser, au moins une partie du milieu non aqueux non siliconé, une partie des monomères acryliques et/ou additionnels, qui constituera, après polymérisation, le squelette insoluble, la totalité du macromonomère (qui constituera les chaînes latérales du polymère) et une partie de l'initiateur de polymérisation. A ce stade d'introduction, le milieu réactionnel forme un milieu relativement homogène.

Le milieu réactionnel est ensuite agité et chauffé jusqu'à une température pour obtenir une polymérisation des monomères et macromonomères. Après un certain temps, le milieu initialement homogène et limpide conduit à une dispersion d'aspect laiteux. On ajoute ensuite un mélange constitué de la partie restante de monomère et de l'initiateur de polymérisation. Après un temps adéquat pendant lequel le mélange est chauffé sous agitation, le milieu se stabilise sous forme d'une dispersion laiteuse, la dispersion comprenant des particules de polymères stabilisés dans le milieu dans lequel elles ont été créées, ladite stabilisation étant due à la présence de chaînes latérales solubles dans ledit milieu.

Il est également possible de préparer la dispersion de particules solides de polymère dans un milieu de polymérisation qui peut ultérieurement être remplacé par un milieu siliconé non aqueux au sens de l'invention.

La dispersion de particules de polymères selon l'invention peut être utilisée dans tout type de composition et notamment dans une composition cosmétique ou pharmaceutique comprenant un milieu cosmétiquement ou pharmaceutiquement acceptable, telle qu'une composition de soin, de nettoyage ou de maquillage pour la peau ou des matières kératiniques, ou une composition capillaire ou une composition solaire.

La dispersion peut être présente à raison de 3 à 95% en poids dans la composition, notamment 4-90% en poids, voire, 20-70% en poids.

De préférence, la composition comprend 0,5 à 25% en poids de matière sèche ou matière active de polymère selon l'invention, notamment 1% à 20% en poids, en particulier 4% à 17% en poids, par exemple 5% à 15% en poids, par rapport au poids total de la composition.

Selon l'application recherchée, la composition peut contenir les adjuvants habituels que l'on incorpore dans des compositions cosmétiques ou pharmaceutiques.

Parmi ces adjuvants, on peut citer les corps gras, et notamment les cires, les huiles, les gommes et/ou les corps gras pâteux, hydrocarbonés et/ou siliconés, et les composés pulvérulents tels que les pigments, les charges et/ou les nacres.

Parmi les cires susceptibles d'être présentes dans la composition selon l'invention, on peut citer, seules ou en mélange, les cires hydrocarbonées telles que la cire d'abeilles ; la cire de Carnauba, de Candellila, d'Ourrury, du Japon, les cires de fibres de liège ou de canne à sucre ; les cires de paraffine, de lignite ; les cires microcristallines ; la cire de lanoline ; la cire de montan ; les ozokérites ; les cires de polyéthylène ; les cires obtenues par synthèse de Fischer-Tropsch ; les huiles hydrogénés, les esters gras et les glycérides concrets à 25°C. On peut également utiliser des cires de silicone, parmi lesquelles on peut citer les alkyls, alcoxys, et/ou esters de polyméthylsiloxane.

Parmi les huiles susceptibles d'être présentes dans la composition selon l'invention, on peut citer, seules ou en mélange, les huiles hydrocarbonées telles que l'huile de paraffine ou de vaseline; le perhydrosqualène ; l'huile d'acara ; l'huile d'amande douce, de calophyllum, de palme, de ricin, d'avocat, de jojoba, d'olive ou de germes de céréales; des esters d'acide lanolique, d'acide oléique, d'acide laurique, d'acide stéarique ; des alcools tels que l'alcool oléique, l'alcool linoléique ou linolénique, l'alcool isostéarique ou l'octyl dodécanol. On peut également citer les huiles siliconées telles que le PDMS, éventuellement phénylées telles que les phényltriméthicones. On peut également citer des huiles volatiles, telles que la cyclotétradiméthylsiloxane, la cyclopentadiméthylsiloxane, la cyclohexadiméthylsiloxane, le méthylhexyldiméthylsiloxane, l'hexaméthyldisiloxane ou les isoparaffines.

Les pigments peuvent être blancs ou colorés, minéraux et/ou organiques. On peut citer parmi les pigments minéraux, les dioxydes de titane, de zirconium ou de cérium, ainsi que les oxydes de zinc, de fer ou de chrome, le bleu ferrique. Parmi les pigments organiques, on peut citer le noir de carbone, et les laques de baryum, strontium, calcium, aluminium.

Les nacres peuvent être choisies parmi le mica recouvert d'oxyde de titane, d'oxyde de fer, de pigment naturel ou d'oxychlorure de bismuth ainsi que le mica titane coloré.

Les charges peuvent être minérales ou de synthèse, lamellaires ou non lamellaires. On peut citer le talc, le mica, la silice, le kaolin, les poudres de nylon et de polyéthylène, le Téflon, l'amidon, le micatitane, le nacre naturelle, le nitrure de bore, les microsphères creuses telles que l'Expancel (Nobel Industrie), le polytrap (Dow Corning) et les microbilles de résine de silicone (Tospearls de Toshiba, par exemple).

La composition peut comprendre en outre tout additif usuellement utilisé dans le domaine cosmétique, tel que des antioxydants, des parfums, des huiles essentielles, des conservateurs, des actifs cosmétiques, des hydratants, des vitamines, des acides gras essentiels, des sphingolipides, des filtres solaires, des tensioactifs, des polymères liposolubles comme les polyalkylènes, notamment le polybutène, les polyacrylates et les polymères siliconés compatibles avec les corps gras. Bien entendu, l'homme du métier veillera à choisir ce ou ces éventuels composés complémentaires, et/ou leur quantité, de manière telle que les propriétés avantageuses de la composition selon l'invention ne soient pas, ou substantiellement pas altérées par l'adjonction envisagée.

Les compositions selon l'invention peuvent se présenter sous toute forme acceptable et usuelle pour une composition cosmétique, hygiénique ou pharmaceutique, et notamment sous forme d'émulsion huile-dans-eau ou eau-dans huile, de lotion, de mousse, de spray.

Parmi les applications préférentiellement visées par la présente invention, on peut plus particulièrement mentionner :

- le domaine des produits capillaires (lavage, soin ou beauté des cheveux), les compositions selon l'invention étant en particulier sous forme d'aérosols, de mousse, de shampooings, d'après-shampooings, de lotions ou de gels coiffants ou traitants, laques ou lotions de mise en forme ou de mise en plis ou encore de fixation ;
- le domaine des produits de maquillage, en particulier pour le maquillage des cils, les compositions étant sous forme de mascara ou de eye-liner ; de rouge à lèvres ou de brillant à lèvres ou de fond de teint, ou encore de fards à joues ou à paupières ;
- le domaine des produits de soin de la peau du corps et du visage, notamment les produits solaires ou autobronzants.

La présente invention a également pour objet un procédé de traitement cosmétique pour le soin, le nettoyage

et/ou le maquillage des matières kératiniques telles que la peau, le cuir chevelu, les cils, les sourcils, les lèvres, les ongles, consistant à appliquer sur lesdites matières kératiniques, une composition telle que définie précédemment.

L'invention va maintenant être décrite plus en détail à la lumière des exemples suivants donnés à titre illustratif et non limitatif.

Les présents exemples illustrent la préparation de polymères conformes à l'invention, aptes à former une dispersion de particules dans un milieu organique considéré.

Dans ces exemples, on détermine, après préparation de ladite dispersion, les masses molaires moyennes en poids (Mw) et en nombre (Mn) du polymère, la température de transition vitreuse du polymère, le taux de matière sèche (ou extrait sec) de la dispersion et la taille des particules de polymères.

Les masses molaires moyennes en poids (Mw) et en nombre (Mn) sont déterminées par chromatographie liquide par perméation de gel (solvant THF, courbe d'étalonnage établie avec des étalons de polystyrène linéaire, détecteur réfractométrique).

La mesure de la température de transition vitreuse (Tg) est effectuée selon la norme ASTM D3418-97, par analyse enthalpique différentielle (DSC "Differential Scanning Calorimetry") sur calorimètre, sur une plage de température comprise entre -100°C et +150°C à une vitesse de chauffe de 10°C/min dans des creusets en aluminium de 150 µl.

La préparation des creusets se fait de la manière suivante : dans un creuset en aluminium de 150 µl on introduit 100 µl de la dispersion obtenue et on laisse le solvant s'évaporer pendant 24h à température ambiante et à 50% d'HR. On renouvelle l'opération puis on introduit le creuset dans le calorimètre Mettler DSC30.

Le taux de matière sèche (ou extrait sec), c'est à dire la teneur en matière non volatile, peut être mesurée de différentes manières : on peut citer par exemple les méthodes par séchage à l'étuve ou les méthodes par séchage par exposition à un rayonnement infrarouge.

De préférence, le taux de matière sèche est mesuré par échauffement de l'échantillon par des rayons infrarouges de 2 µm à 3,5 µm de longueur d'onde. Les substances contenues dans la composition qui possèdent une pression de vapeur élevée s'évaporent sous l'effet de ce rayonnement. La mesure de la perte de poids de l'échantillon permet de déterminer l'extrait sec de la composition. Ces mesures sont réalisées au moyen d'un dessiccateur à infrarouges commercial LP16 de chez Mettler. Cette technique est parfaitement décrite dans la documentation de l'appareil fournie par Mettler.

Le protocole de mesure est le suivant : on étale environ 1g de la composition sur une coupelle métallique. Celle-ci, après introduction dans le dessiccateur, est soumise à une consigne de température de 120°C pendant une heure. La masse humide de l'échantillon, correspondant à la masse initiale et la masse sèche de l'échantillon, correspondant à la masse après exposition au rayonnement, sont mesurées au moyen d'une balance de précision.

Le taux de matière sèche est calculée de la manière suivante :

$$\text{Extrait Sec} = 100 \times (\text{masse sèche} / \text{masse humide}).$$

Les tailles de particules peuvent être mesurées par différentes techniques : on peut citer en particulier les techniques de diffusion de la lumière (dynamiques et statiques), les méthodes par compteur Coulter, les mesures par vitesse de sédimentation (reliée à la taille via la loi de Stokes) et la microscopie. Ces techniques permettent de mesurer un diamètre de particules et pour certaines d'entre elles une distribution granulométrique.

De préférence, les tailles et les distributions de tailles des particules des compositions selon l'invention, sont mesurées par diffusion statique de la lumière au moyen d'un granulomètre commercial de type MasterSizer 2000 de chez Malvern. Les données sont traitées sur la base de la théorie de diffusion de Mie. Cette théorie, exacte pour des particules isotropes, permet de déterminer dans le cas de particules non sphériques, un diamètre « effectif » de particules. Cette théorie est notamment décrite dans l'ouvrage de Van de Hulst, H.C., "Light Scattering by Small Particles," Chapitres 9 et 10, Wiley, New York, 1957. La composition est caractérisée par son diamètre « effectif » moyen en volume D[4,3], défini de la manière suivante :

$$D[4,3] = \frac{\sum_i V_i \cdot d_i}{\sum_i V_i}$$

où $V_i$ représente le volume des particules de diamètre effectif $d_i$. Ce paramètre est notamment décrit dans la documentation technique du granulomètre.

Les mesures sont réalisées à 25 °C, sur une dispersion de particules diluée, obtenue à partir de la composition de la manière suivante : 1) dilution d'un facteur 100 avec de l'eau, 2) homogénéisation de la solution, 3) repos de la solution durant 18 heures, 4) récupération du surnageant homogène blanchâtre.
Le diamètre « effectif » est obtenu en prenant un indice de réfraction de 1,33 pour l'eau et un indice de réfraction moyen de 1,42 pour les particules.

**EXEMPLE 1**

Cet exemple illustre la préparation d'un polymère formant une dispersion de particules dans un solvant carboné, ledit polymère étant obtenu par polymérisation d'acrylate de méthyle et le macromonomère correspondant à un copolymère polyéthylène/polybutylène (Kraton L-1253).
Dans un réacteur de 1 litre, on charge 200 g d'heptane, 200 g d'isododécane, 28 g d'acrylate de méthyle, 12 g de macromonomère du type oligomère polyéthylène/polybutylène à monoterminaison méthacrylate, de Mw = 4000 (Kraton L-1253) et 3,2 g de tertio butyl peroxy-2-éthylhexanoate (Trigonox 21S).
On agite et on chauffe le mélange réactionnel à température ambiante à 90°C en 1 heure. Après 15 minutes à 90°C, on observe un changement d'aspect du milieu réactionnel, qui passe d'un aspect transparent à un aspect laiteux. On maintient le chauffage sous agitation pendant 15 minutes supplémentaires puis on ajoute goutte à goutte pendant 1 heure un mélange constitué par 160 g d'acrylate de méthyle et 2 g de Trigonox 21S.
On laisse ensuite le chauffage pendant 4 heures à 90°C puis on distille l'heptane du milieu réactionnel. A l'issue de cette opération de distillation, on obtient une dispersion de particules de polymère stable dans l'isododécane.
Les caractéristiques du polymère et des particules formées par ledit polymère sont les suivantes :

- Masse moléculaire poids Mw : 119212
- Masse moléculaire nombre Mn : 31896
- Indice de polydispersité (Mw/Mn)= 3,74
- Transition vitreuse :10°C par DSC Mettler
- Extrait sec : 49,8% dans l'isododécane, réalisé par thermobalance ;
- Granulométrie : 46 nm avec polydispersité de 0,05 réalisée sur Malvern Autosizer Lo-C à 25°C.

Le macromonomère représente 6% en poids par rapport au poids du polymère.
La stabilité de la dispersion obtenue est mise en évidence par la mise en oeuvre du protocole de stabilité suivant : dans un tube à hémolyse, on place 8 ml de la dispersion réalisée et on centrifuge à 4000 tours/min pendant 15 minutes à l'aide d'une centrifugeuse Jouan C100-S5. Au bout de 15 minutes, on constate qu'il n'y a pas de déphasage ce qui démontre que la dispersion est stable.

**EXEMPLE 2**

Cet exemple illustre la préparation d'un polymère formant une dispersion de particules dans un solvant carboné, ledit polymère étant obtenu par polymérisation d'acrylate de méthoxyéthyle et le macromonomère correspondant à un copolymère polyéthylène/polybutylène (Kraton L-1253).
Dans un réacteur de 500 ml, on charge 75 g d'heptane, 50 g d'isododécane, 12,75 g d'acrylate de méthoxyéthyle et 2,25 g de macromonomère du type copolymère de polyéthylène/polybutylène à monoterminaison méthacrylate de Mw = 4000 (Kraton L-1253) et 0,8 g de tertio butyl peroxy-2-éthylhexanoate (Trigonox 21S).
On agite et on chauffe le mélange réactionnel à température ambiante à 90°C en 1 heure. Après 15 minutes à 90°C, on observe un changement d'aspect du milieu réactionnel, qui passe d'un aspect transparent à un aspect laiteux. On maintient le chauffage sous agitation pendant 15 minutes supplémentaires puis on ajoute goutte à goutte pendant 1 heure un mélange constitué par 35 g d'acrylate de méthoxyéthyle et 0,5 g de Trigonox 21S.
On laisse ensuite le chauffage pendant 4 heures à 90°C puis on distille l'heptane du milieu réactionnel. A l'issue de cette opération de distillation, on obtient une dispersion de particules de polymère ainsi préparé stable dans l'isododécane.
Les caractéristiques du polymère et des particules formées par ledit polymère sont les suivantes :

- Masse moléculaire poids Mw= 71200
- Masse moléculaire nombre Mn = 19300
- Indice de polydispersité (Mw/Mn) = 3,7
- Transition vitreuse : -40°C par DSC Mettler ;
- Extrait sec : 56,4 % dans l'isododécane, réalisé par thermobalance ;
- Granulométrie : 91,4 nm avec polydispersité de 0,05 réalisée sur Malvern Autosizer Lo-C à 25°C

Le macromonomère représente 4,5% en poids par rapport au poids du polymère.

Après la mise en oeuvre du protocole de stabilité conformément à l'exemple 1, on constate que la dispersion obtenue est stable.

## EXEMPLE 3

Cet exemple illustre la préparation d'un polymère formant une dispersion de particules dans un solvant carboné, ledit polymère étant obtenu par polymérisation d'acrylate de méthyle et le macromonomère correspondant à un copolymère polyéthylène/polybutylène (Kraton L-1253).

Dans un réacteur de 1 litre, on charge 200 g d'heptane, 200 g d'isononyl isononanoate, 28 g d'acrylate de méthyle, 12 g de macromonomère du type copolymère de polyéthylène/polybutylène à monoterminaison méthacrylate (Kraton L-1253) et 3,2 g de tertio butyl peroxy-2-éthylhexanoate (Trigonox 21S).

On agite et on chauffe le mélange réactionnel à température ambiante à 90°C en 1 heure. Après 15 minutes à 90°C, on observe un changement d'aspect du milieu réactionnel, qui passe d'un aspect transparent à un aspect laiteux. On maintient le chauffage sous agitation pendant 15 minutes supplémentaires puis on ajoute goutte à goutte pendant 1 heure un mélange constitué par 160 g d'acrylate de méthyle et 2 g de Trigonox 21S.

On laisse ensuite le chauffage pendant 4 heures à 90°C puis on distille l'heptane du milieu réactionnel. A l'issue de cette opération de distillation, on obtient une dispersion de particules de polymère ainsi préparé stable dans l'isononyl isononanoate.

Les caractéristiques du polymère et des particules formées par ledit polymère sont les suivantes :

- Masse moléculaire poids Mw=98909
- Masse moléculaire nombre Mn=25731
- Indice de polydispersité (Mw/Mn)= 3,84
- Transition vitreuse : 12°C par DSC Mettler ;
- Extrait sec théorique : 50 % dans l'isononyl isononanoate
- Granulométrie : 220 nm avec polydispersité de 0,04 réalisée sur Malvern Autosizer Lo-C à 25°C.

Le macromonomère représente 6% en poids par rapport au poids du polymère.

Après la mise en oeuvre du protocole de stabilité conformément à l'exemple 1, on constate que la dispersion obtenue est stable.

## EXEMPLE 4

Cet exemple illustre la préparation d'un polymère formant une dispersion de particules dans un solvant carboné, ledit polymère étant obtenu par polymérisation d'acrylate de méthyle et le macromonomère correspondant à un copolymère polyéthylène/polybutylène (Kraton L-1253).

Dans un réacteur de 1 litre, on charge 200 g d'heptane, 200 g d'alkyl benzoate en C12-15 plus connu sous le nom de Finsolv TN, 28 g d'acrylate de méthyle, 12 g de macromonomère du type copolymère de polyéthylène/polybutylène à monoterminaison méthacrylate (Kraton L-1253) et 3,2 g de tertio butyl peroxy-2-éthylhexanoate (Trigonox 21S).

On agite et on chauffe le mélange réactionnel à température ambiante à 90°C en 1 heure. Après 15 minutes à 90°C, on observe un changement d'aspect du milieu réactionnel, qui passe d'un aspect transparent à un aspect laiteux. On maintient le chauffage sous agitation pendant 15 minutes supplémentaires puis on ajoute goutte à goutte pendant 1 heure un mélange constitué par 160 g d'acrylate de méthyle et 2 g de Trigonox 21S.

On laisse ensuite le chauffage pendant 4 heures à 90°C puis on distille l'heptane du milieu réactionnel. A l'issue de cette opération de distillation, on obtient une dispersion de particules de polymère ainsi préparé stable dans l' alkyl benzoate en C12-15 ( Finsolv TN )

Les caractéristiques du polymère et des particules formées par ledit polymère sont les suivantes :

- Masse moléculaire poids Mw=93984
- Masse moléculaire nombre Mn=29923
- Indice de polydispersité (Mw/Mn)= 3.14
- Transition vitreuse : 12°C par DSC Mettler ;
- Extrait sec théorique : 50 % dans l' alkyl benzoate en C12-15 ( Finsolv TN )
- Granulométrie : 50 nm avec polydispersité de 0,04 réalisée sur Malvern Autosizer Lo-C à 25°C.

Le macromonomère représente 6% en poids par rapport au poids du polymère.

Après la mise en oeuvre du protocole de stabilité conformément à l'exemple 1, on constate que la dispersion obtenue est stable.

**EXEMPLE 5**

Cet exemple illustre la préparation d'un polymère formant une dispersion de particules dans un solvant carboné, ledit polymère étant obtenu par polymérisation d'acrylate de méthyle et le macromonomère correspondant à un copolymère polyéthylène/polybutylène (Kraton L-1253).

Dans un réacteur de 1 litre, on charge 200 g d'heptane, 200 g d'octyldodécanol, 28 g d'acrylate de méthyle, 12 g de macromonomère du type copolymère de polyéthylène/polybutylène à monoterminaison méthacrylate (Kraton L-1253) et 3,2 g de tertio butyl peroxy-2-éthylhexanoate (Trigonox 21S).

On agite et on chauffe le mélange réactionnel à température ambiante à 90°C en 1 heure. Après 15 minutes à 90°C, on observe un changement d'aspect du milieu réactionnel, qui passe d'un aspect transparent à un aspect laiteux. On maintient le chauffage sous agitation pendant 15 minutes supplémentaires puis on ajoute goutte à goutte pendant 1 heure un mélange constitué par 160 g d'acrylate de méthyle et 2 g de Trigonox 21S.

On laisse ensuite le chauffage pendant 4 heures à 90°C puis on distille l'heptane du milieu réactionnel. A l'issue de cette opération de distillation, on obtient une dispersion de particules de polymère ainsi préparé stable dans l'octyldodécanol.

Les caractéristiques du polymère et des particules formées par ledit polymère sont les suivantes :

- Masse moléculaire poids Mw=95375
- Masse moléculaire nombre Mn=20109
- Indice de polydispersité (Mw/Mn)= 4,74
- Transition vitreuse : 12°C par DSC Mettler ;
- Extrait sec théorique : 50 % dans l'octyldodécanol

Le macromonomère représente 6% en poids par rapport au poids du polymère.

Après la mise en oeuvre du protocole de stabilité conformément à l'exemple 1, on constate que la dispersion obtenue est stable.

**EXEMPLE 6**

Cet exemple illustre la préparation d'un polymère formant une dispersion de particules dans un solvant carboné, ledit polymère étant obtenu par polymérisation d'acrylate de méthyle et le macromonomère correspondant à un copolymère polyéthylène/polybutylène (Kraton L-1253).

Dans un réacteur de 1 litre, on charge 200 g d'heptane, 200 g de diisostéaryl malate, 28 g d'acrylate de méthyle, 12 g de macromonomère du type copolymère de polyéthylène/polybutylène à monoterminaison méthacrylate (Kraton L-1253) et 3,2 g de tertio butyl peroxy-2-éthylhexanoate (Trigonox 21S).

On agite et on chauffe le mélange réactionnel à température ambiante à 90°C en 1 heure. Après 15 minutes à 90°C, on observe un changement d'aspect du milieu réactionnel, qui passe d'un aspect transparent à un aspect laiteux. On maintient le chauffage sous agitation pendant 15 minutes supplémentaires puis on ajoute goutte à goutte pendant 1 heure un mélange constitué par 160 g d'acrylate de méthyle et 2 g de Trigonox 21S.

On laisse ensuite le chauffage pendant 4 heures à 90°C puis on distille l'heptane du milieu réactionnel. A l'issue de cette opération de distillation, on obtient une dispersion de particules de polymère ainsi préparé stable dans le diisostéaryl malate.

Les caractéristiques du polymère et des particules formées par ledit polymère sont les suivantes :

- Masse moléculaire poids Mw=120224
- Masse moléculaire nombre Mn=32665
- Indice de polydispersité (Mw/Mn)= 4,74
- Transition vitreuse : 12°C par DSC Mettler ;
- Extrait sec théorique : 50% dans le diisostéaryl malate

Le macromonomère représente 6% en poids par rapport au poids du polymère.

Après la mise en oeuvre du protocole de stabilité conformément à l'exemple 1, on constate que la dispersion obtenue est stable.

**EXEMPLE 7**

Cet exemple illustre la préparation d'un polymère formant une dispersion de particules dans un solvant carboné, ledit polymère étant obtenu par polymérisation d'acrylate de méthyle et de méthacrylate de méthyle et le macromonomère correspondant à un copolymère polyéthylène/polybutylène (Kraton L-1253).

Dans un réacteur de 1 litre, on charge 200 g d'heptane, 200 g d'isododécane, 24 g d'acrylate de méthyle, 16 g de macromonomère du type copolymère de polyéthylène/polybutylène à monoterminaison méthacrylate (Kraton L-1253) et 3,2 g de tertio butyl peroxy-2-éthylhexanoate (Trigonox 21S).

On agite et on chauffe le mélange réactionnel à température ambiante à 90°C en 1 heure. Après 15 minutes à 90°C, on observe un changement d'aspect du milieu réactionnel, qui passe d'un aspect transparent à un aspect laiteux. On maintient le chauffage sous agitation pendant 15 minutes supplémentaires puis on ajoute goutte à goutte pendant 1 heure un mélange constitué par 120 g d'acrylate de méthyle, 40 g de méthacrylate de méthyle et 2 g de Trigonox 21S. On laisse ensuite le chauffage pendant 4 heures à 90°C puis on distille l'heptane du milieu réactionnel. A l'issue de cette opération de distillation, on obtient une dispersion de particules de polymère ainsi préparé stable dans l'isododécane.

Les caractéristiques du polymère et des particules formées par ledit polymère sont les suivantes :

- Masse moléculaire poids Mw=156900
- Masse moléculaire nombre Mn=19200
- Indice de polydispersité (Mw/Mn)= 8,15
- Transition vitreuse : 35°C par DSC Mettler ;
- Extrait sec théorique : 53,2% dans l'isododécane

Le macromonomère représente 8% en poids par rapport au poids du polymère.

Après la mise en oeuvre du protocole de stabilité conformément à l'exemple 1, on constate que la dispersion obtenue est stable.

**EXEMPLE 8**

Cet exemple illustre la préparation d'un polymère formant une dispersion de particules dans un solvant carboné, ledit polymère étant obtenu par polymérisation d'acrylate de méthyle, d'acide acrylique et le macromonomère correspondant à un copolymère polyéthylène/polybutylène (Kraton L-1253).

Dans un réacteur de 1 litre, on charge 200 g d'heptane, 200 g d'isododécane, 28 g d'acrylate de méthyle, 12 g de macromonomère du type copolymère de polyéthylène/polybutylène à monoterminaison méthacrylate (Kraton L-1253) et 3,2 g de tertio butyl peroxy-2-éthylhexanoate (Trigonox 21S).

On agite et on chauffe le mélange réactionnel à température ambiante à 90°C en 1 heure. Après 15 minutes à 90°C, on observe un changement d'aspect du milieu réactionnel, qui passe d'un aspect transparent à un aspect laiteux. On maintient le chauffage sous agitation pendant 15 minutes supplémentaires puis on ajoute goutte à goutte pendant 1 heure un mélange constitué par 150 g d'acrylate de méthyle, 10 g d'acide acrylique et 2 g de Trigonox 21S. On laisse ensuite le chauffage pendant 4 heures à 90°C puis on distille l'heptane du milieu réactionnel. A l'issue de cette opération de distillation, on obtient une dispersion de particules de polymère ainsi préparé stable dans l'isododécane.

Les caractéristiques du polymère et des particules formées par ledit polymère sont les suivantes :

- Masse moléculaire poids Mw=143639
- Masse moléculaire nombre Mn=23965
- Indice de polydispersité (Mw/Mn)= 5,99
- Extrait sec théorique : 51,3 % dans l'isododécane
- Granulométrie : 48 nm avec polydispersité de 0,04 réalisée sur Malvern Autosizer Lo-C à 25°C.

Le macromonomère représente 6% en poids par rapport au poids du polymère.

Après la mise en oeuvre du protocole de stabilité conformément à l'exemple 1, on constate que la dispersion obtenue est stable.

**EXEMPLE 9**

Cet exemple illustre la préparation d'un polymère formant une dispersion de particules dans un solvant carboné, ledit polymère étant obtenu par polymérisation d'acrylate de méthyle, de méthacrylate de 2-hydroxyéthyle, le

macromonomère correspondant à un copolymère polyéthylène/polybutylène (Kraton L-1253).

Dans un réacteur de 1 litre, on charge 200 g d'heptane, 200 g d'isododécane, 24 g d'acrylate de méthyle, 16 g de macromonomère du type copolymère de polyéthylène/polybutylène à monoterminaison méthacrylate (Kraton L-1253) et 3,2 g de tertio butyl peroxy-2-éthylhexanoate (Trigonox 21S).

On agite et on chauffe le mélange réactionnel à température ambiante à 90°C en 1 heure. Après 15 minutes à 90°C, on observe un changement d'aspect du milieu réactionnel, qui passe d'un aspect transparent à un aspect laiteux. On maintient le chauffage sous agitation pendant 15 minutes supplémentaires puis on ajoute goutte à goutte pendant 1 heure un mélange constitué par 120 g d'acrylate de méthyle, 40 g de méthacrylate de 2-hydroxyéthyle et 2 g de Trigonox 21S.

On laisse ensuite le chauffage pendant 4 heures à 90°C puis on distille l'heptane du milieu réactionnel. A l'issue de cette opération de distillation, on obtient une dispersion de particules de polymère ainsi préparé stable dans l'isododécane.

Les caractéristiques du polymère et des particules formées par ledit polymère sont les suivantes :

- Masse moléculaire poids Mw=178500
- Masse moléculaire nombre Mn=29700
- Indice de polydispersité (Mw/Mn)= 6,01
- Extrait sec théorique : 49,8% dans l'isododécane

Le macromonomère représente 8% en poids par rapport au poids du polymère.

Après la mise en oeuvre du protocole de stabilité conformément à l'exemple 1, on constate que la dispersion obtenue est stable.

## EXEMPLE 10

Cet exemple illustre la préparation d'un polymère formant une dispersion de particules dans un solvant carboné, ledit polymère étant obtenu par polymérisation d'acrylate de méthyle, de méthacrylate de diméthylaminoéthyle et le macromonomère correspondant à un copolymère polyéthylène/polybutylène (Kraton L-1253).

Dans un réacteur de 1 litre, on charge 200 g d'heptane, 200 g d'isododécane, 24 g d'acrylate de méthyle, 16 g de macromonomère du type copolymère de polyéthylène/polybutylène à monoterminaison méthacrylate (Kraton L-1253) et 3,2 g de tertio butyl peroxy-2-éthylhexanoate (Trigonox 21S).

On agite et on chauffe le mélange réactionnel à température ambiante à 90°C en 1 heure. Après 15 minutes à 90°C, on observe un changement d'aspect du milieu réactionnel, qui passe d'un aspect transparent à un aspect laiteux. On maintient le chauffage sous agitation pendant 15 minutes supplémentaires puis on ajoute goutte à goutte pendant 1 heure un mélange constitué par 140 g d'acrylate de méthyle, 20 g de méthacrylate de diméthylaminoéthyle et 2 g de Trigonox 21S.

On laisse ensuite le chauffage pendant 4 heures à 90°C puis on distille l'heptane du milieu réactionnel. A l'issue de cette opération de distillation, on obtient une dispersion de particules de polymère ainsi préparé stable dans l'isododécane.

Les caractéristiques du polymère et des particules formées par ledit polymère sont les suivantes :

- Extrait sec théorique : 51,6% dans l'isododécane

Le macromonomère représente 8% en poids par rapport au poids du polymère.

Après la mise en oeuvre du protocole de stabilité conformément à l'exemple 1, on constate que la dispersion obtenue est stable.

## Exemple 11 : Composition de mascara

On a préparé un mascara ayant la composition suivante :

| | |
|---|---|
| Cire d'abeille | 8 g |
| Cire de paraffine | 3 g |
| Cire de carnauba | 6 g |
| Hectorite modifiée par du chlorure de di-stéaryl di-méthyl benzyl ammonium (Bentone® 38V d'Elementis) | 5,3 g |
| Carbonate de propylène | 1,7 g |

(suite)

| | |
|---|---|
| Charge | 1 g |
| pigments | 5 g |
| Dispersion de polymère de l'exemple 4 | 10 g MS* |
| Isododécane | qsp 100 g |

*MS : matière sèche

Le mascara, après application sur les cils, est jugé très satisfaisant.

**Exemple 12 : Stick de rouge à lèvres**

| La composition de rouge à lèvres suivante est préparée : | |
|---|---|
| Cire de polyéthylène | 15 % |
| Dispersion de polymère de l'exemple 3 | 10 % en MS |
| Polyisobutène hydrogéné (Parléam de Nippon Oil Fats) | 26 % |
| Pigments | 8.6 % |
| Isododécane | qsp 100% |

La composition obtenue après application sur les lèvres présente de bonnes propriétés cosmétiques.

**Exemple 13 : Fond de teint E/H**

On prépare une composition de fond de teint comprenant les composés suivants :

| | | |
|---|---|---|
| Phase A | Cetyl Dimethicone copolyol (ABIL EM 90 de la société GOLDSCHMIDT) | 3 g |
| | Succinate d'isostéaryl diglycéryle (IMWITOR 780K de la société CONDEA) | 0,6 g |
| | Isododécane | 18,5 g |
| | Pigments (oxydes de fer et oxydes de titane hydrophobes) | 10 g |
| | Dispersion de polymère de l'exemple 2 | 8g en MS |
| | Poudre de polyamide (NYLON-12 de Dupont de Nemours) | 8 g |
| | Parfum | qs |
| Phase B | Eau | qsp 100 g |
| | Sulfate de magnésium | 0,7 g |
| | Conservateur (Methylparaben) | qs |
| Phase C | Eau | 2 g |
| | Conservateur (Diazolinyl urée) | qs |

La composition obtenue présente de bonnes propriétés cosmétiques.

**Exemple 14**

On prépare une poudre compactée ayant la composition suivante :

*Composition A :*

- Talc 30 g
- Oxychlorure de bismuth 10 g

- Stéarate de zinc 4 g
- Poudre de Nylon 20 g
- Dispersion de l'exemple 1 5 g

*Composition B* :

- Oxydes de fer 2 g
- Huile de vaseline 6 g

La poudre est obtenue de la façon suivante : on broie la composition A dans un broyeur de type KENWOOD pendant environ 5 minutes sous faible agitation, on ajoute la composition B et on broie l'ensemble environ 2 minutes à la même vitesse, puis 3 minutes à une vitesse plus rapide. On tamise ensuite la préparation sur un tamis de 0,16 mm, puis on compacte ce mélange dans des coupelles.
On obtient une poudre compactée présentant de bonnes propriétés cosmétiques.
La composition obtenue est aisée et agréable à appliquer. On constate que le film ne migre pas dans les ridules de la peau, même après avoir été porté pendant plusieurs heures.

**Exemple 15 : gel pour le visage**

On prépare la composition suivante :

. isopropyl palmitate 10 g
. vaseline (cire) 5 g
. hectorite modifiée (argile) 0,15 g
. ozokérite (cire) 5 g
. septaoléate de sorbitane oxyéthyléné (40OE) 5 g
. dispersion de l'exemple 4 (25% de matière sèche) 75 g

On obtient un gel ayant de bonnes propriétés cosmétiques.

**Exemple 16 : huile de soin**

On prépare la composition suivante :

. dispersion de l'exemple 3 (25% de matière sèche) 70 g
. huile de jojoba 15 g
. huile de soja 15 g

On obtient une huile de soin qui peut être appliquée sur le corps ou le visage.

## Revendications

1. Dispersion, dans un milieu organique non aqueux non siliconé, de particules constituées d'au moins un polymère acrylique comprenant un squelette insoluble dans ledit milieu, et une partie soluble dans ledit milieu constituée de chaînes latérales liées de manière covalente audit squelette, ledit polymère étant susceptible d'être obtenu par polymérisation radicalaire dans ledit milieu :

    - d'au moins un monomère acrylique, pour former ledit squelette insoluble ; et
    - d'au moins un macromonomère carboné comportant un groupe terminal apte à réagir pendant la polymérisation pour former les chaînes latérales, ledit macromonomère ayant une masse moléculaire en poids supérieure ou égale à 200 et représentant 1 à 18% en poids du polymère.

2. Dispersion selon la revendication 1, dans laquelle le milieu organique non aqueux non siliconé est constitué d'au

moins 50% en poids d'au moins un composé liquide non aqueux non siliconé choisi parmi :

- les composés liquides non aqueux non siliconés ayant un paramètre de solubilité global selon l'espace de solubilité de Hansen inférieur ou égal à 18 $(MPa)^{1/2}$ ;
- les monoalcools ayant un paramètre de solubilité global selon l'espace de solubilité de Hansen inférieur ou égal à 20 $(MPa)^{1/2}$ ; et
- leurs mélanges.

**3.** Dispersion selon la revendication 2, dans laquelle le composé liquide non aqueux non siliconé ayant un paramètre de solubilité global selon l'espace de solubilité de Hansen inférieur ou égal à 18 $(MPa)^{1/2}$ est choisi parmi les huiles naturelles ou synthétiques, carbonées, hydrocarbonées, fluorées, seules ou en mélange ; les alcanes linéaires, ramifiés et/ou cycliques, éventuellement volatils; les esters et notamment les esters linéaires, ramifiés ou cycliques, ayant plus de 6 atomes de carbone; les cétones et notamment les cétones ayant plus de 6 atomes de carbone; les éthers et notamment les éthers ayant plus de 6 atomes de carbone.

**4.** Dispersion selon la revendication 2, dans laquelle les monoalcools ayant un paramètre de solubilité global selon l'espace de solubilité de Hansen inférieur ou égal à 20 $(MPa)^{1/2}$ sont choisis parmi les monoalcools gras aliphatiques ayant 6 à 30 atomes de carbone, la chaîne hydrocarbonée ne comportant pas de groupement de substitution, et notamment l'alcool oléique, le décanol, le dodécanol, l'octadécanol et l'alcool linoléique.

**5.** Dispersion selon l'une des revendications précédentes, dans laquelle le monomère acrylique est choisi parmi, seul ou en mélange, les monomères suivants, ainsi que leurs sels :

- (i) les (méth)acrylates de formule :

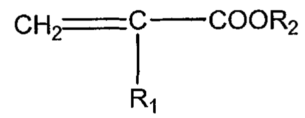

dans laquelle :

- $R_1$ désigne un atome d'hydrogène ou un groupe méthyle ;
- $R_2$ représente un groupe choisi parmi :
  - un groupe alkyle linéaire ou ramifié, comprenant de 1 à 6 atomes de carbone, ledit groupe pouvant comporter dans sa chaîne un ou plusieurs hétéroatomes choisis parmi O, N et S; et/ou pouvant comporter un ou plusieurs substituants choisis parmi -OH, les atomes d'halogène (F, Cl, Br, I) et
  - NR'R" avec R' et R" identiques ou différents choisis parmi les alkyles linéaires ou ramifiés en C1-C4; et/ou pouvant être substitué par au moins un groupe polyoxyalkylène, notamment polyoxyéthylène et/ou polyoxypropylène, ledit groupe polyoxyalkylène étant constitué par la répétition de 5 à 30 motifs oxyalkylène;
  - un groupe alkyle cyclique comprenant de 3 à 6 atomes de carbone, ledit groupe pouvant comporter dans sa chaîne un ou plusieurs hétéroatomes choisis parmi O, N et S, et/ou pouvant comporter un ou plusieurs substituants choisis parmi OH et les atomes d'halogène (F, Cl, Br, I);

- (ii) les (méth)acrylamides de formule :

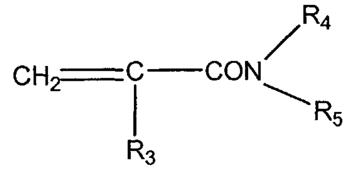

dans laquelle :

- $R_3$ désigne un atome d'hydrogène ou un groupe méthyle ;
- $R_4$ et $R_5$, identiques ou différents, représentent un atome d'hydrogène ou un groupe alkyle, linéaire ou ramifié, comportant de 1 à 6 atomes de carbone, pouvant comporter un ou plusieurs substituants choisis parmi -OH, les atomes d'halogène (F, Cl, Br, I) et -NR'R" avec R' et R" identiques ou différents choisis parmi les alkyles linéaires ou ramifiés en C1-C4;ou
- $R_4$ représente un atome d'hydrogène et $R_5$ représente un groupe 1,1-diméthyl-3-oxobutyle.

- (iii) des monomères à insaturation(s) éthylénique(s) comprenant au moins une fonction acide carboxylique, phosphorique ou sulfonique, tel que l'acide acrylique, l'acide méthacrylique, l'acide acrylamidopropanesulfonique, et leurs sels;

**6.** Dispersion selon la revendication 5, dans laquelle le monomère acrylique est choisi parmi les (méth)acrylates de méthyle, d'éthyle, de propyle, de butyle, d'isobutyle; les (méth)acrylates de méthoxyéthyle ou d'éthoxyéthyle; le méthacrylate de trifluoroéthyle; le méthacrylate de diméthylaminoéthyle, le méthacrylate de diéthylaminoéthyle, le méthacrylate de 2-hydroxypropyle, le méthacrylate de 2-hydroxyéthyle, l'acrylate de 2-hydroxypropyle, l'acrylate de 2-hydroxyéthyle ; le diméthylaminopropylméthacrylamide; et leurs sels.

**7.** Dispersion selon l'une des revendications précédentes, dans laquelle le polymère acrylique est susceptible d'être obtenu par polymérisation radicalaire d'un ou plusieurs monomères acryliques en mélange avec un ou plusieurs monomères additionnels vinyliques non acryliques.

**8.** Dispersion selon la revendication 7, dans laquelle les monomères additionnels vinyliques sont choisis parmi :

- les esters de vinyle de formule :

$$R_6\text{-COO-CH=CH}_2$$

  dans laquelle $R_6$ représente un groupe alkyle linéaire ou ramifié, comprenant de 1 à 6 atomes, ou un groupe alkyle cyclique comportant de 3 à 6 atomes de carbone et/ou un groupe aromatique, par exemple de type benzénique, anthracénique, et naphtalénique ;
- des monomères à insaturation(s) éthylénique(s) comprenant au moins une fonction acide carboxylique, phosphorique ou sulfonique, tel que l'acide crotonique, l'anhydride maléique, l'acide itaconique, l'acide fumarique, l'acide maléique, l'acide styrènesulfonique, l'acide vinylbenzoïque, l'acide vinylphosphorique et les sels de ceux-ci ;
- les monomères à insaturation(s) éthylénique(s) comprenant au moins une fonction amine tertiaire, tel que la 2-vinylpyridine, la 4-vinylpyridine,
  et leurs mélanges.

**9.** Dispersion selon l'une des revendications précédentes, dans laquelle le macromonomère constituant après réaction les chaînes latérales du polymère selon l'invention, comporte en bout de chaîne un groupe terminal polymérisable choisi parmi un groupe vinyle ou (méth)acryloyloxy (acrylate ou méthacrylate).

**10.** Dispersion selon l'une des revendications précédentes, dans laquelle le macromonomère présente une masse moléculaire en poids (Mw) allant de 200 à 100 000, de préférence de 300 à 50 000, notamment 500 à 20 000, préférentiellement de 800 à 10000, voire de 1000 à 6000.

**11.** Dispersion selon l'une des revendications précédentes, dans laquelle le macromonomère est choisi

- (i) les homopolymères et les copolymères d'acrylate ou de méthacrylate d'alkyle linéaire ou ramifié en C6-C22, présentant un groupe terminal choisi parmi les groupes vinyle ou (méth)acryloyloxy parmi lesquels on peut citer :

  - les macromonomères de poly(acrylate d'éthyl-2 hexyle) à extrémité monoacrylate ou monométhacrylate;
  - les macromonomères de poly(acrylate de dodécyle) ou de poly(méthacrylate de dodécyle) à extrémité monoacrylate ou monométhacrylate;
  - les macromonomères de poly(acrylate de stéaryle) ou de poly(méthacrylate de stéaryle) à extrémité monoacrylate ou monométhacrylate.

- (ii) les polyoléfines présentant un groupe terminal à insaturation éthylénique de type vinyle ou (méth)acryloyloxy, parmi lesquelles :

  - les macromonomères de polyéthylène, les macromonomères de polypropylène, les macromonomères de polyisobutylène, les macromonomères de polybutadiène; tous ces macromonomères étant à extrémité monoacrylate ou monométhacrylate;
  - les macromonomères de polyisoprène à extrémité monoacrylate ou monométhacrylate;
  - les macromonomères de poly(éthylène/butylène)-polyisoprène à extrémité monoacrylate ou monométhacrylate;
  - les macromonomères de copolymère polyéthylène/polypropylène ou de copolymère polyéthylène/polybutylène, à extrémité monoacrylate ou monométhacrylate.

**12.** Dispersion selon l'une des revendications précédentes, dans laquelle le macromonomère est présent dans le polymère dans une proportion de 2 à 16% en poids, préférentiellement de 4 à 15% en poids, et notamment de 6 à 12% en poids, voire de 8% à 10% en poids, par rapport au poids total dudit polymère.

**13.** Dispersion selon l'une des revendications précédentes, dans laquelle la masse moléculaire en poids (Mw) du polymère est comprise entre 10 000 et 300 000, notamment entre 20 000 et 200 000, mieux encore entre 25 000 et 150 000.

**14.** Dispersion selon l'une des revendications précédentes, dans laquelle les particules de polymères sont des particules solides de taille moyenne allant de 10 à 400 nm, de préférence de 20 à 200 nm.

**15.** Dispersion selon l'une des revendications précédentes, présentant un taux de matière sèche (ou extrait sec) de 40% à 70% en poids de matière sèche, notamment de 45 à 65% en poids.

**16.** Composition cosmétique ou pharmaceutique comprenant, dans un milieu cosmétiquement ou pharmaceutiquement acceptable, une dispersion selon l'une des revendications précédentes.

**17.** Composition selon la revendication 16, dans laquelle la dispersion est présente à raison de 3 à 95% en poids dans la composition, notamment 4-90% en poids, voire, 20-70% en poids.

**18.** Composition selon l'une des revendications 16 à 17, comprenant, en outre, des corps gras choisis parmi les cires, les huiles, les gommes, les corps gras pâteux, hydrocarbonés ou siliconés; et/ou des composés pulvérulents tels que les pigments, les charges et/ou les nacres; et/ou des antioxydants, des parfums, des huiles essentielles, des conservateurs, des actifs cosmétiques, des hydratants, des vitamines, des acides gras essentiels, des sphingolipides, des filtres solaires, des tensioactifs, des polymères liposolubles comme les polyalkylènes, notamment le polybutène, les polyacrylates et les polymères siliconés compatibles avec les corps gras.

**19.** Composition selon l'une des revendications 16 à 18, se présentant sous la forme d'une composition de soin, de nettoyage ou de maquillage pour la peau ou les matières kératiniques, d'une composition capillaire, d'une composition solaire.

**20.** Procédé de traitement cosmétique pour le soin, le nettoyage et/ou le maquillage des matières kératiniques telles que la peau, le cuir chevelu, les cils, les sourcils, les lèvres, les ongles, consistant à appliquer sur lesdites matières kératiniques, une composition telle que définie à l'une des revendications 16 à 19.